# EUROPÄISCHE PATENTSCHRIFT

(11) **EP 2 146 773 B1**
(45) Veröffentlichungstag und Bekanntmachung des Hinweises auf die Patenterteilung: **09.11.2011**
(21) Anmeldenummer: 08734428.9
(22) Anmeldetag: 22.03.2008
(51) Int. Cl.: A61M 39/12, F16L 33/00

(54) **Verfahren zur Herstellung einer Rohr- und Schlauchverbindung**
Method for producing a pipe and hose connection
Procédé de production d'un raccord de tube et de tuyau flexible

(30) Priorität: 19.04.2007 DE 202007005754 U
(43) Veröffentlichungstag der Anmeldung: 27.01.2010
(73) Patentinhaber: Völker, Manfred, 63825 Blankenbach (DE)
(72) Erfinder: Völker, Manfred, 63825 Blankenbach (DE)
(74) Vertreter: Flosdorff, Jürgen
(86) Internationale Anmeldenummer: PCT/DE2008/000498
(87) Internationale Veröffentlichungsnummer: WO 2008/128496

(56) Entgegenhaltungen:
- DE-A1- 10 262 036
- DE-A1- 19 528 160
- GB-A- 1 030 485
- NL-C- 68 987
- US-A- 5 358 012
- US-A- 5 722 150
- US-B1- 6 206 048

## Beschreibung

Die Erfindung betrifft ein Verfahren zur Herstellung einer Rohr- und Schlauchverbindung.

Derartige Rohr- und Schlauchverbindungen werden unter anderem in der medizinischen Intensivtherapie eingesetzt, beispielsweise um Dialysegeräte mit Flüssigkeiten zu versorgen oder verbrauchte Flüssigkeiten abzuführen. In der DE 195 28 160 C2 sind Rohr- und Schlauchverbindungen der betrachteten Art offenbart.

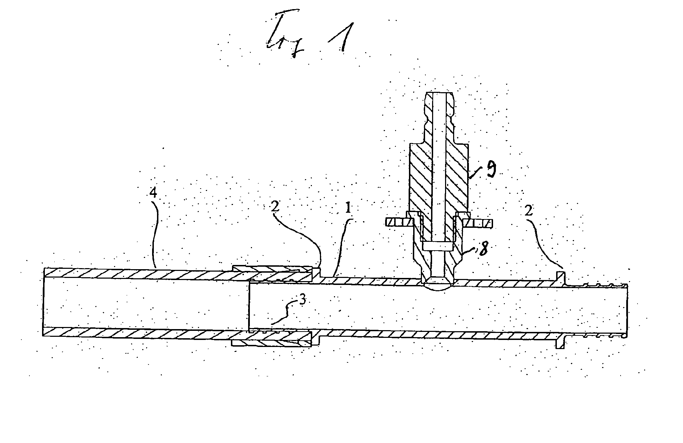

Eine druck- und thermisch beständige Rohr- und Schlauchverbindung herkömmlicher Art ist in Figur 1 abgebildet. Ein Rohr 1 aus einem starren Material, vorzugsweise aus Metall, ist mit einem seitlich abzweigenden. Rohrstutzen 8 versehen, in den eine Schnellkupplung 9 eingeschraubt ist. Diese seitliche Abzweigung der durch das Rohr 1 fließenden Flüssigkeit ist hier nicht von Interesse.

Das Rohr 1 enthält an beiden Endbereichen jeweils einen Flansch 2, an den sich ein Rohrstutzen 3 anschließt, der mit voneinander beabstandeten ringförmigen Vorsprüngen versehen ist. Auf diese Rohrstutzen 3 ist jeweils ein Schlauch 4 aufpressbar, und zwar bis zu dem Flansch 2, der als Anschlag für den Aufpressvorgang dient.

Diese Ausbildung hat einen erheblichen spanenden Aufwand und Materialverlust bei der Ausbildung der Flansche zur Folge.

Der vorliegenden Erfindung liegt die Aufgabe zugrunde, diesen hohen Materialeinsatz und den Aufwand bei der spanenden Bearbeitung zu verringern.

Diese Aufgabe wird erfindungsgemäß durch die Merkmale des Anspruchs 1 gelöst.

Vorteilhafte Ausgestaltungen der Erfindung sind in den Unteransprüchen gekennzeichnet.

Die Erfindung sieht vor, das der Rohrstutzen von dem anschließenden Rohr durch eine Ringnut getrennt ist, in die ein Anschlag für den Schlauch lösbar einsetzbar ist. Dieser Anschlag wird beim Aufpressvorgang des Schlauches in die Nut eingesetzt und nach Herstellung der Rohr- und Schlauchverbindung wieder entfernt.

Hierdurch ist erreicht, dass das Rohr mit verringertem Materialverlust und geringstem spanenden Aufwand aus einem Rohrhalbzeug herstellbar ist. Dabei kann die Schlauchverbindung an einem oder an beiden Endabschnitten des Rohrs erfolgen.

Mit großem Vorteil wird vorgeschlagen, das der Anschlag ein offener Ring ist, der seitlich in die Nut einschiebbar ist. Der Ring hat dabei einen solchen Außendurchmesser, das er über den Außenumfang des Rohres übersteht, so dass der Ring ebenso wie der Flansch beim Stand der Technik als Anschlag beim Aufpressen des Schlauches wirkt. Nach dem Aufpressen wird der Ring aus der Nut entfernt und steht für einen weiteren Aufpressvorgang zur Verfügung.

Der Ring hat eine mittige Aussparung mit einem kreisbogenförmigen Rand, dessen Durchmesser im wesentlichen dem Außendurchmesser des Nutbodens entspricht. Die Öffnung des Rings kann eine Breite haben, die mit dem Außendurchmesser des Nutbodens übereinstimmt, so dass der Ring glatt in die Nut einschiebbar ist.

Die Öffnung des Rings kann aber auch etwas kleiner als der Außendurchmesser des Nutbodens sein, wobei dann die Öffnung des Rings beim Einschieben in die Nut elastisch aufgeweitet wird, wodurch der Ring nach dem Zurückfedern in der Nut gehalten ist.

Wie oben erwähnt, kann das Rohr an beiden Enden jeweils einen Rohrstutzen mit einer angrenzenden Nut zur Befestigung zweier Schläuche aufweisen. Dabei kann das Rohr geradlinig ausgebildet sein oder beispielsweise eine U-Form haben.

Weitere Einzelheiten der Erfindung ergeben sich aus der nachfolgenden Beschreibung mit Bezug auf die Zeichnungen. Dabei zeigen:
- Fig. 2: eine Ausführungsform der Erfindung, bei der das Rohr an beiden Enden mit Schläuchen verbunden wird, beim Aufpressen eines Schlauches;
- Fig. 3: einen T-Verbinder mit einem aufgepresstem Schlauch und
- Fig. 4: einen 90-Grad-Verbinder mit einem aufgepressten Schlauch.

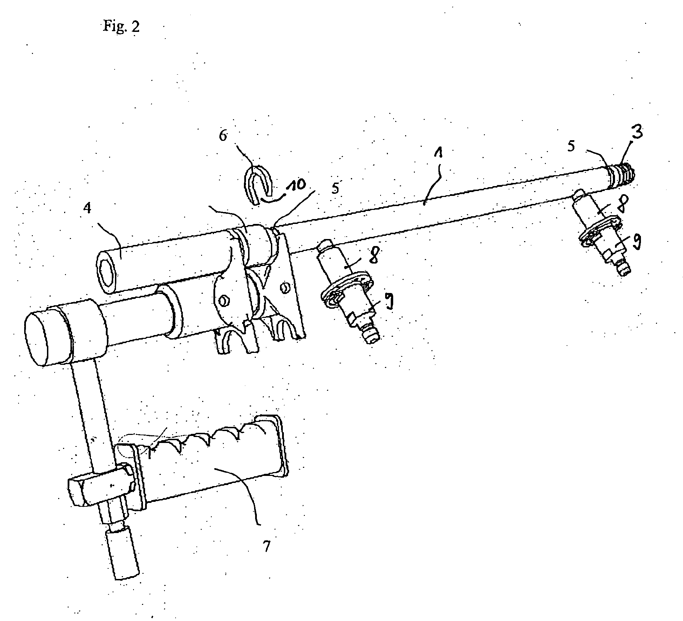

Fig. 2 zeigt ein geradliniges Rohr 1 mit zwei seitlichen Abzweigungen 8, 9. In dem Rohr 1 sind an beiden Endbereichen ringförmige Nuten 5 ausgebildet, vorzugsweise eingefräst, denen Rohrstutzen 3 mit ringförmigen Vorsprüngen folgen.

Auf den linken Rohrstutzen 3 wird in der Darstellung der Fig. 2 mittels eines dem Fachmann bekannten Werkzeugs 7 ein Schlauch 4 aufgepresst, nachdem in die linke Nut 5 ein offener Ring 6 lösbar eingesetzt worden ist. Der Ring 6 hat eine Öffnung 10, deren Breite mit dem Außendurchmesser des Nutbodens übereinstimmt, so dass sich der Ring 6 mit seiner kreisbogenförmigen Innenkontur dicht an den Nutboden anlegt. Der Ring 6, dessen Wandstärke mit der Nutbreite im wesentlichen übereinstimmt, sitzt fest in der Nut, so dass er ebenso als Anschlag für den aufgepressten Schlauch 4 dient wie beim Stand der Technik der Flansch 2.

Zum Aufpressen des zweiten Schlauchs auf den rechten Endabschnitt wird der Ring 6 aus der linken Nut 5 entnommen und in die rechte Nut 5 eingesetzt.

Durch die Ausbildung der Nuten kann das Rohr 1 aus einem Rohrhalbzeug kleineren Außendurchmessers als beim Stand der Technik hergestellt werden.

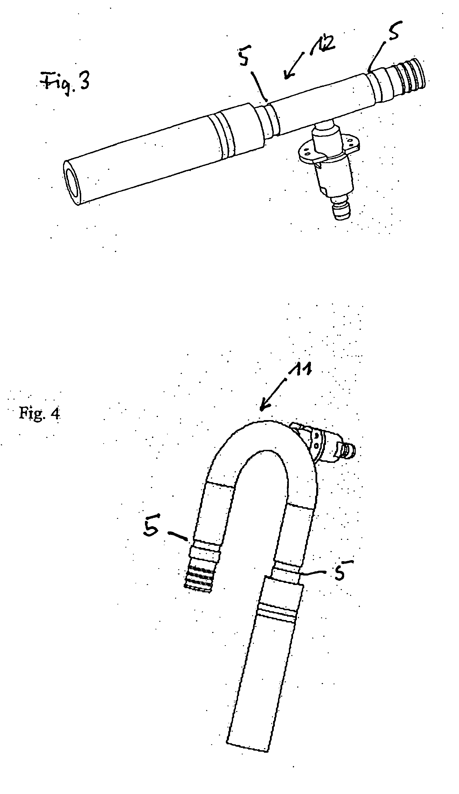

Fig. 3 zeigt einen T-Verbinder 12, während in Fig. 4 ein 90-Grad-Verbinder 11 dargestellt ist, die jeweils mit zwei Nuten 5 zum Einsetzen des lösbaren Rings 6 versehen sind.

## Patentansprüche

1. Verfahren zur Herstellung einer Rohr- und Schlauchverbindung,
**gekennzeichnet durch** folgende Schritte:
- Ausbilden wenigstens einer ringförmigen Nut (5) an einem Rohr (1), **durch** die ein mit dem Rohr (1) einstückiger Rohrstutzen (3) von dem Rohr (1) getrennt wird,
- lösbares Einsetzen eines Anschlags (6) für den Schlauch (4) in die Nut (5),
- Aufpressen des Schlauchs (4) auf den Rohrstutzen (3) und Entfernen des Anschlags (6).

2. Verfahren nach Anspruch 1,
**dadurch gekennzeichnet,**
**dass** die wenigstens eine ringförmige Nut (5) eingefräst ist.

3. Verfahren nach Anspruch 1 oder 2,
**dadurch gekennzeichnet,**
**dass** als Anschlag (6) ein offener Ring verwendet wird, der seitlich in die Nut (5) einschiebbar ist.

4. Verfahren nach Ansprüch 3,
**dadurch gekennzeichnet,**
**dass** der Ring (6) eine mittige Aussparung mit einem kreisbogenförmigen Rand hat, dessen Durchmesser im wesentlichen mit dem Außendurchmesser des Nutbodens übereinstimmt.

5. Verfahren nach einem der Ansprüche 3 oder 4,
**dadurch gekennzeichnet,**
**dass** der Ring (6) eine Öffnung mit einer Breite hat, die mit dem Außendurchmesser des Nutbodens übereinstimmt.

6. Verfahren nach einem der Ansprüche 3 oder 4,
**dadurch gekennzeichnet,**
**dass** der Ring (6) eine Öffnung mit einer Breite hat, die etwas kleiner ist als der Außendurchmesser des Nutbodens.

7. Verfahren nach einem der Ansprüche 1 bis 6,
**dadurch gekennzeichnet,**
**dass** der Rohrstutzen (3) mit voneinander beabstandeten ringförmigen Vorsprüngen versehen wird.

8. Verfahren nach einem der Ansprüche 1 bis 7,
**dadurch gekennzeichnet,**
**dass** an beiden Endbereichen des Rohres jeweils eine ringförmige Nut ausgebildet wird, die jeweils einen Rohrstutzen (3) von dem Rohr trennt.

9. Verfahren nach einem der Ansprüche 1 bis 8,
**dadurch gekennzeichnet,**
**dass** das Rohr (1) geradlinig ausgebildet ist.

10. Verfahren nach einem der Ansprüche 1 bis 8,
**dadurch gekennzeichnet,**
**dass** das Rohr (1) eine U-Form hat.

## Claims

1. A method of producing a pipe and hose connection, **characterised by** the following steps:
- forming at least one annular groove (5) on a pipe (i), by which a pipe socket (3) integral with the pipe (1) is separated from the pipe (1),
- releasably inserting an abutment (6) for the hose (4) into the groove (5),
- pressing the hose (4) onto the pipe socket (3) and removing the abutment (6).

2. A method as claimed in claim 1, **characterised in that** the at least one annular grove (5) is formed by milling.

3. A method as claimed in claim 1 or 2, **characterised in that** an open ring, which may be slid laterally into the groove (5), as used as the abutment (6)

4. A method as claimed in claim 3, **characterised in that** the ring (6) has a central opening with a circular arcuate edge, the diameter of which substantially corresponds to the external diameter of the base of the groove.

5. A method as claimed in one of claims 3 or 4, **characterised in that** the ring (6) has an opening with a breadth, which corresponds to the external diameter of the base of the groove.

6. A method as claimed in one of claims 3 or 4, **characterised in that** the ring (6) has an opening with a breadth which is somewhat smaller than the external diameter of the base of the groove.

7. A method as claimed in any one of claims 1 to 6, **characterised in that** the pipe socket (3) is provided with annular projections spaced from one an another.

8. A method as claimed in any one of claims 1 to 7, **characterised in that** formed on both end regions of the pipe there is a respective annular groove, which separates a respective pipe socket (3) from the pipe.

9. A method as claimed in any one of claims 1 to 8, **characterised in that** the pipe (1) is straight.

10. A method as claimed in any one of claims 1 to 8, **characterised in that** the pipe (1) is of U shape.

## Revendications

1. Procédé de fabrication d'un raccord de tube et de tuyau,
**caractérisé par** les étapes suivantes consistant à :
- réaliser au moins une rainure annulaire (5) sur un tube (1), ladite rainure permettant de séparer du tube (1) une tubulure (3) faisant partie intégrante du tube (1),
- insérer de manière amovible une butée (6) pour le tuyau (4) dans la rainure (5),
- presser le tuyau (4) sur la tubulure (3) et retirer la butée (6).

2. Procédé selon la revendication 1,
**caractérisé en ce que**
la ou les rainures annulaires (5) sont fraisées.

3. Procédé selon la revendication 1 ou 2,
**caractérisé en ce**
**qu'**une bague ouverte pouvant être introduite latéralement dans la rainure (5) est utilisée comme butée (6).

4. Procédé selon la revendication 3,
**caractérisé en ce que**
la bague (6) présente un évidement central doté d'un bord en forme d'arc de cercle dont le diamètre concorde sensiblement avec le diamètre extérieur du fond de la rainure.

5. Procédé selon l'une quelconque des revendications 3 ou 4,
**caractérisé en ce que**
la bague (6) présente une ouverture dotée d'une largeur coïncidant avec le diamètre extérieur du fond de la rainure.

6. Procédé selon l'une quelconque des revendications 3 ou 4,
**caractérisé en ce que**
la bague (6) présente une ouverture dotée d'une largeur sensiblement inférieure au diamètre extérieur du fond de la rainure.

7. Procédé selon l'une quelconque des revendications 1 à 6,
**caractérisé en ce que**
la tubulure (3) est pourvue de parties en saillie annulaires distantes les unes des autres.

8. Procédé selon l'une quelconque des revendications 1 à 7,
**caractérisé en ce que**
respectivement une rainure annulaire séparant respectivement une tubulure (3) du tube est formée sur les deux zones d'extrémité du tube.

9. Procédé selon l'une quelconque des revendications 1 à 8,
**caractérisé en ce que**
le tube (1) présente une forme rectiligne.

10. Procédé selon l'une quelconque des revendications 1 à 8,
**caractérisé en ce que**
le tube (1) présente une forme de U.
